(19) Europäisches Patentamt
European Patent Office
Office européen des brevets

(11) Veröffentlichungsnummer : **0 021 391**
**B1**

(12) **EUROPÄISCHE PATENTSCHRIFT**

(45) Veröffentlichungstag der Patentschrift : **01.09.82**

(51) Int. Cl.³ : **C 07 F 9/38**, A 61 K 31/66// C07F9/40

(21) Anmeldenummer : **80103520.5**

(22) Anmeldetag : **24.06.80**

# (54) Phosphonoameisensäurehydrazide, Verfahren zu ihrer Herstellung sowie ihre Verwendung als Arzneimittel.

(30) Priorität : **03.07.79 DE 2926799**

(43) Veröffentlichungstag der Anmeldung : **07.01.81 (Patentblatt 81/01)**

(45) Bekanntmachung des Hinweises auf die Patenterteilung : **01.09.82 Patentblatt 82/35**

(84) Benannte Vertragsstaaten : **AT BE CH DE FR GB IT LI NL SE**

(56) Entgegenhaltungen :
**AU D 4 268 378**
**DE A 2 728 685**
**JOURNAL FÜR PRAKTISCHE CHEMIE, Band 320, Heft 2, 1978, Leipzig, H. GROSS et al. : « Alfa-Substituierte Phosphonate. XXX(1). Entkalkylierung von Phosphonsäureestern mit labilen funktionellen Gruppen mittels Trimethylsilylbromid », Seiten 344-50**

(73) Patentinhaber : **BAYER AG**
**Zentralbereich Patente, Marken und Lizenzen**
**D-5090 Leverkusen 1, Bayerwerk (DE)**

(72) Erfinder : **Oediger, Hermann, Dr.**
**Roggendorfstrasse 51**
**D-5000 Köln 80 (DE)**
Erfinder : **Lieb, Folker, Dr.**
**Alfred-Kubin-Strasse 1**
**D-5090 Leverkusen (DE)**
Erfinder : **Streissle, Gert, Dr.**
**Am Hochsitz 17**
**D-5600 Wuppertal (DE)**

**EP 0 021 391 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

# Phosphonoameisensäurehydrazide, Verfahren zu ihrer Herstellung sowie ihre Verwendung als Arzneimittel

Die vorliegende Erfindung betrifft neue Phosphonoameisensäurehydrazide, Verfahren zu ihrer Herstellung sowie ihre Verwendung als Arzneimittel, insbesondere als antivirale mittel in der Human- und Tiermedizin.

Man kennt bereits antivirale Mittel, beispielsweise gegenüber Herpes simplex-Viren wirkende Mittel aus der Stoffklasse der Nucleoside, wie das 5-Jod-2'-desoxyuridin [M. Negwer, Organisch-chemische Arzneimittel und ihre Synonyma, Seite 187, Nr. 1017 ; Akademie-Verlag, Berlin 1978]. Sie haben jedoch häufig unerwünschte Nebenwirkungen, wie mutagene, teratogene oder immunsuppressive Effekte.

Darüber hinaus ist beispielsweise aus der Reihe des Aminoadamantans das N-(1-Adamantyl)-2-(2-dimethyl-aminoethoxy)-acetamid bekannt geworden [DOS 1 941 218]. Es ist jedoch im Vergleich zu bekannten antiviralen Mitteln nur schwach wirksam.

In letzter Zeit wurde auch die Phosphonoameisensäure als antivirales Mittel bekannt [DOS 2 728 685]. Aus EP-A 3 008 sind außerdem Phosphonoameisensäureamide bekannt, die jedoch keine antiviralen Wirkungen aufweisen.

Es wurde nun gefunden, daß die neuen Phosphonameisensäurehydrazide der Formel (I)

$$R\text{-}NH\text{-}NH\text{-}CO\text{-}\overset{O}{\overset{\|}{P}}(OH)_2 \qquad (I)$$

in der R für Wasserstoff oder Alkyl mit 1-6 C-Atomen steht, und/oder physiologisch verträgliche Salze derselben starke antiviralische Eigenschaften aufweisen.

Weiterhin wurde gefunden, daß man die Phosphonoameisensäurehydrazide der Formel (I) erhält, wenn man entweder

a) einen Phosphonoameisensäurealkylester der Formel (II)

$$R_2O\text{-}CO\text{-}\overset{O}{\overset{\|}{P}}(O\text{-}R_1)_2 \qquad (II)$$

in welcher $R_1$ für Alkyl steht und $R_2$ für Alkyl und Aryl stehen, mit einem Trialkylsilylhalogenid der Formel (III)

$$(R_3)_3Si\text{—}Hal \qquad (III)$$

in der $R_3$ für einen Alkylrest und Hal für Brom oder Jod stehen, umsetzt, die erhaltene Verbindung der Formel (IV)

$$R_2O\text{-}CO\text{-}\overset{O}{\overset{\|}{P}}(O\text{-}Si(R_3)_3)_2 \qquad (IV)$$

in der $R_2$ und $R_3$ die obige Bedeutung haben, mit Wasser umsetzt und die erhaltene Verbindung der Formel (V)

$$R_2O\text{-}CO\text{-}\overset{O}{\overset{\|}{P}}(OH)_2 \qquad (V)$$

in der $R_2$ die obige Bedeutung hat, in Form ihrer Salze, beispielsweise ihrer Alkalisalze, mit einem Hydrazin der Formel (VI)

$$R\text{—}NH\text{—}NH_2 \qquad (VI)$$

in der R die obige Bedeutung hat, umsetzt, oder

b) einen Phosphonoameisensäureester der Formel (VII)

$$R_2O\text{-}CO\text{-}\overset{O}{\overset{\|}{P}}(O\text{-}benzyl)_2 \qquad (VII)$$

in welcher $R_2$ die obige Bedeutung hat, mit Wasserstoff in Gegenwart eines Edelmetallkatalysators die Benzylgruppe hydrogenolytisch abspaltet, die erhaltene Verbindung der Formel (V), in der $R_2$ die obige Bedeutung hat, in Form ihrer Salze, beispielsweise ihrer Alkalisalze, mit einem Hydrazin der Formel (VI) umsetzt, oder

c) Phosphonoameisensäurealkylester der Formel (II) mit einem Hydrazin umsetzt, die erhaltene Verbindung der Formel (VIII)

$$R\text{-}NH\text{-}NH\text{-}CO\text{-}\overset{O}{\overset{\|}{P}}(O\text{-}R_1)_2 \qquad \text{(VIII)}$$

in der R und $R_1$ obige Bedeutungen haben, mit einem Silylierungsreagens umsetzt und die erhaltene Verbindung der Formel (IX)

$$(R_3)_3Si\text{-}\overset{R}{\overset{|}{N}}\text{-}NH\text{-}CO\text{-}\overset{O}{\overset{\|}{P}}(OR)_1)_2 \qquad \text{(IX)}$$

in der R, $R_1$ und $R_3$ obige Bedeutungen haben, mit einem Trialkylsilylhalogenid der Formel (III) umsetzt und die erhaltene Verbindung der Formel (X)

$$(R_3)_3Si\text{-}\overset{R}{\overset{|}{N}}\text{-}NH\text{-}CO\text{-}\overset{O}{\overset{\|}{P}}(O\text{-}Si(R_3)_3)_2 \qquad \text{(X)}$$

in der R und $R_3$ die obige Bedeutung haben, mit Wasser umsetzt.

Überraschenderweise zeigen die erfindungsgemäßen Phosphonoameisensäurehydrazide eine erheblich höhere und/oder breiter antivirale Wirksamkeit als die aus dem Stand der Technik bekannten Stoffe. Die erfindungsgemäßen Stoffe stellen somit eine Bereicherung der Pharmazie dar.

Verwendet man beim Reaktionsablauf a) als Ausgangsstoffe Dimethoxyphosphonoameisensäuremethylester und Trimethylsilyljodid, zur Neutralisation Natronlauge und Hydrazin, so kann der Reaktionsablauf durch Reaktionsschema [A] wiedergegeben werden.

(Siehe Formeln Seite 4)

Reaktionsschema [A]

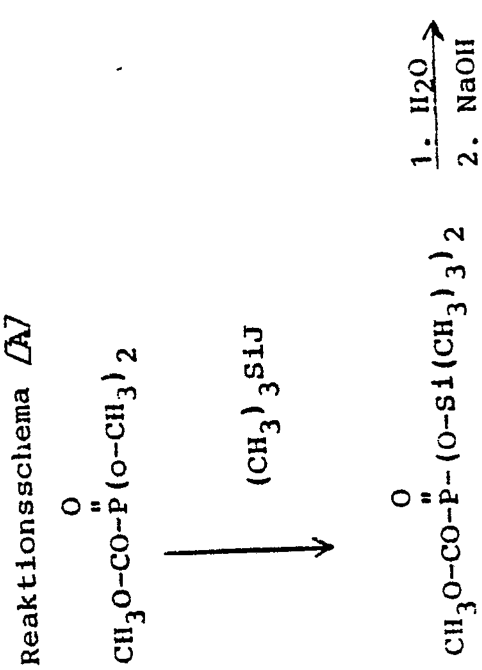


Reaktionsschema [B]

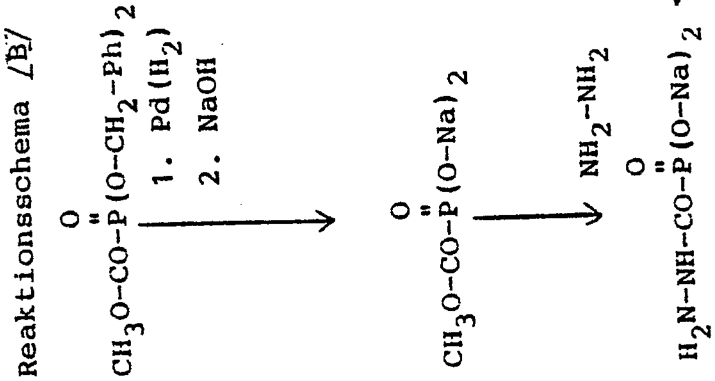


Reaktionsschema [C]

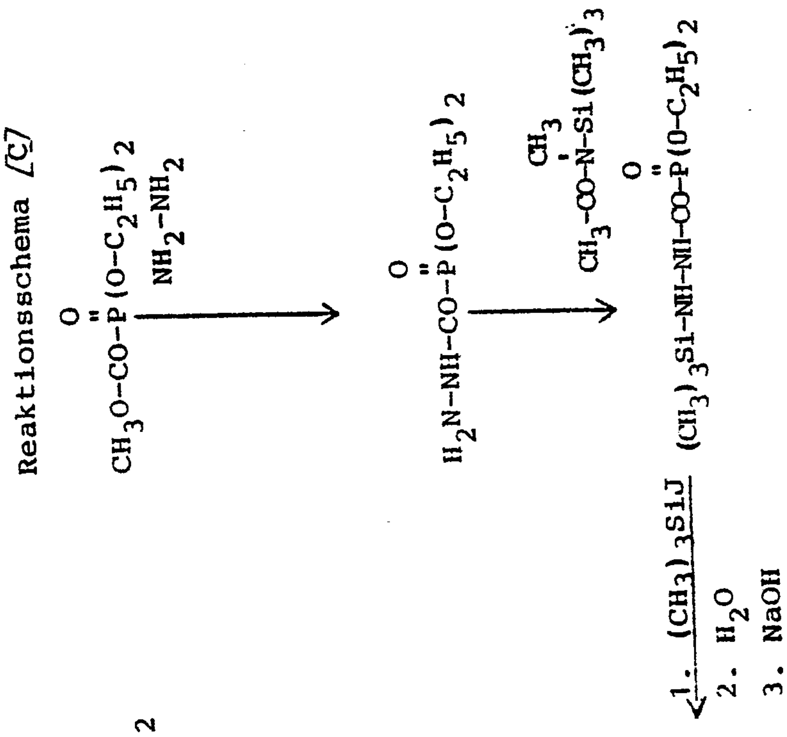

Die als Ausgangsstoffe verwendeten Phosphonoameisensäureester sind bekannt [Houben-Weyl, Methoden der organischen Chemie, Bd. XII/1, Seite 456].

In der Formel (II) stehen vorzugsweise $R_1$ für Alkyl mit 1 bis 4 Kohlenstoffatomen, insbesondere für Methyl und Ethyl, $R_2$ für Alkyl mit 1 bis 2 Kohlenstoffatomen, insbesondere für Methyl und für Aryl, insbesondere für gegebenenfalls substituiertes Phenyl.

Beispielsweise seien genannt:
Dimethoxyphosphonoameisensäuremethylester,
Diethoxyphosphonoameisensäuremethylester,
Dimethoxyphosphonoameisensäureethylester,
Diethoxyphosphonoameisensäureethylester.
Dimethoxyphosphonoameisensäurephenylester
Diethoxyphosphonoameisensäurephenylester.

Die als Ausgangsstoffe verwendeten Trialkylsilylhalogenide sind ebenfalls bekannt.

In der Formel (III) stehen vorzugsweise $R_3$ für Alkyl mit 1 bis 4 Kohlenstoffatomen, wobei die Alkylgruppen auch untereinander verschieden sein können, insbesondere für Methyl, Hal für Brom oder Jod, insbesondere für Jod, wenn $R_1$ für Ethyl steht.

Dabei ist es nicht erforderlich, beispielsweise die Trialkylsilyljodide als solche einzusetzen. Es genügt, diese in Gegenwart der Phosphonoameisensäureester der allgemeinen Formel (II) aus den entsprechenden Trialkylsilylchloriden und Natriumjdodid herzustellen.

Als Verdünnungsmittel kommen für die Reaktionspartner inerte organische Lösungsmittel in Frage. Geeignete Lösungsmittel sind Kohlenwasserstoffe wie Halogenkohlenwasserstoffe, beispielsweise Tetrachlormethan, oder aromatische Kohlenwasserstoffe, beispielsweise Toluol oder Alkancarbonsäurenitrile mit 2 bis 4 Kohlenstoffatomen, beispielsweise Aceto- oder Propionitril, vorzugsweise Acetonitril.

Das erfindungsgemäße Verfahren wird in einem Temperaturbereich zwischen + 10 °C und + 80 °C, vorzugsweise zwischen + 20 °C und + 70 °C durchgeführt.

Im allgemeinen setzt man 1 Mol der Verbindung (II) mit 2,0 bis 3,0, vorzugsweise mit 2,0 bis 2,2 Mol der Verbindung (III) um. Ein größerer Überschuß an (III) schadet nicht, ist jedoch unwirtschaftlich.

Die Reaktionsdauer ist von der Temperatur und den eingesetzten Ausgangsverbindungen abhängig und liegt zwischen 15 Minuten und 3 Stunden.

Entsprechend dem Reaktionsschema [A] wird die Verbindung der allgemeinen Formel (IV) mit Wasser umgesetzt.

Das erfindungsgemäße Verfahren wird in einem Temperaturbereich zwischen Null + 40 °C, vorzugsweise zwischen + 20 und + 30 °C durchgeführt.

Im allgemeinen setzt man 1 Mol der Verbindung (IV) mit mindestens 2 Mol Wasser, zweckmäßig mit einem größeren Überschuß von etwa 30 Mol Wasser um.

Die Reaktionsdauer ist von der Temperatur und von der Struktur der Silylgruppe abhängig und liegt im allgemeinen zwischen 1 Minute und 10 Minuten.

Die erhaltene Verbindung der allgemeinen Formel (V) wird im allgemeinen in Form ihrer Alkalisalze, beispielsweise als Lithium- oder Natriumsalz, etwa durch Versetzen mit einer ausreichenden Menge wäßriger Lithium- oder Natronlauge und Eindampfen isoliert.

Entsprechend dem Reaktionsschema [A] wird die Verbindung der allgemeinen Formel (V) in Form ihrer Alkalisalze mit einem Hydrazin der allgemeinen Formel (VI) umgesetzt.

In der Formel (VI) stehen vorzugsweise R für Wasserstoff und Alkyl mit 1 bis 6 Kohlenstoffatomen, insbesondere für Wasserstoff und Alkyl mit 1 bis 4 Kohlenstoffatomen.

Beispielsweise seien genannt:
Hydrazin,
Methylhydrazin,
Ethylhydrazin,
Propylhydrazin,
Butylhydrazin,
Hexylhydrazin.

Dabei kann das Hydrazin als solches oder beispielsweise auch in Form seiner wäßrigen Lösung eingesetzt werden. Als Verdünnungsmittel kommen vor allem Wasser und/oder mit Wasser mischbare und für die Reaktionspartner inerte organische Lösungsmittel in Frage. Geeignete Lösungsmittel sind vor allem aliphatische Alkohole mit 1 bis 4 Kohlenstoffatomen, beispielsweise Methanol oder Ethanol.

Das erfindungsgemäße Verfahren wird in einem Temperaturbereich von + 10 bis + 80 °C, vorzugsweise zwischen + 20 und + 60 °C durchgeführt.

Im allgemeinen setzt man 1 Mol der Verbindung (V), beispielsweise in Form ihres Alkalisalzes, mit 1 bis 5 Mol, vorzugsweise mit 2 bis 4 Mol eines Hydrazins um.

Die Reaktionsdauer ist von der Temperatur abhängig und liegt im allgemeinen zwischen 2 Stunden und 8 Stunden.

Verwendet man beim Reaktionsablauf b) als Ausgangsstoff Dibenzyloxyphosphonoameisensäurem ethylester, so kann der Reaktionsablauf durch Reaktionsschema [B] wiedergegeben werden.

Alternativ wird entsprechend dem Reaktionsschema [B] in der ersten Stufe des Verfahrens ein Aralkyloxyphosphonoameisensäureester der allgemeinen Formel (VII) in Gegenwart eines Edelmetalls

hydrogenolytisch mit Wasserstoff gespalten.

Die als Ausgangsstoffe verwendeten Aralkyloxyphosphonoameisensäureester sind weitgehend nicht bekannt.

Sie können aber nach bekannten Methoden hergestellt werden, beispielsweise aus Trisaralkylphosphit mit Halogenameisensäureestern [Houben-Weyl : Methoden der organischen Chemie, Bd. XII/1, Seite 456, Georg Thieme Verlag, Stuttgart 1963].

In der Formel (VII) steht $R_2$ vorzugsweise für Alkyl mit 1 bis 2 Kohlenstoffatomen, insbesondere für Methyl und für Aryl, insbesondere für gegebenenfalls substituiertes Phenyl.

Beispielsweise seien genannt :

Dibenzyloxyphosphonoameisensäuremethylester,
Dibenzyloxyphosphonoameisensäureethylester,
Dibenzyloxyphosphonoameisensäurephenylester.

Als Edelmetall eignet sich besonderes fein verteiltes Palladium.

Als Verdünnungsmittel kommen vor allem für die Reaktionspartner inerte organische Lösungsmittel in Frage. Geeignete Lösungsmittel sind vor allem aliphatische Alkohole mit 1 bis 4 Kohlenstoffatomen, wie Methanol oder Ethanol, aliphatische Ester mit 3 bis 8 Kohlenstoffatomen, wie Essigsäureethylester oder Gemische der genannten Lösungsmittel.

Das erfindungsgemäße Verfahren wird in einem Temperaturbereich von + 20 °C bis + 150 °C, vorzugsweise zwischen + 30 °C bis + 100 °C durchgeführt.

Die Reaktion kann bei Normaldruck oder auch bei erhöhtem Druck durchgeführt werden. Im letzteren Fall arbeitet man im allgemeinen bei Drucken zwischen 2 und 100 bar, vorzugsweise zwischen etwa 5 und 50 bar.

Der erhaltene Phosphonoameisensäureester der Formel (V) wird beispielsweise in Form seiner Salze, wie seiner Alkalisalze, etwa durch Versetzen mit einer ausreichenden Menge wäßriger Lithium- oder Natronlauge und Eindampfen isoliert und entsprechend dem Reaktionsschema [B] mit einem Hydrazin entsprechend der dritten Stufe des Reaktionsschemas [A] umgesetzt.

Verwendet man beim Reaktionsablauf c) als Ausgangsstoff Diethoxyphosphonoameisensäuremethylester, als Silylierungsreagenz N-Methyl-trimethylsilylacetamid und zur Spaltung des Phosphonoesters Trimethyljodsilan, so kann der Rekationsablauf durch das Reaktionsschema [C] wiedergegeben werden.

Alternativ entsprechend dem Reaktionsschema [C] in der ersten Stufe des Verfahrens ein Dialkoxyphosphonoameisensäureester der allgemeinen Formel (II) mit einem Hydrazin der Formel (VI) umgesetzt.

In der Formel (II) stehen $R_1$ für Alkyl mit 1 bis 4 Kohlenstoffatomen, vorzugsweise für Methyl und Ethyl, insbesondere für Ethyl, $R_2$ für Alkyl mit 1 bis 4 Kohlenstoffatomen, vorzugsweise für Methyl und Ethyl, insbesondere für Methyl, für Aryl, insbesondere für gegebenenfalls substituiertes Phenyl. Es ist nicht notwendig, wasserfreies Hydrazin einzusetzen. Es kann auch beispielsweise in Form einer wäßrigen Lösung eingesetzt werden. Als Verdünnungsmittel kommen vor allem mit Wasser mischbare und für die Reaktionspartner inerte organische Lösungsmittel in Frage. Geeignete Lösungsmittel sind vor allem aliphatische Alkohole mit 1 bis 4 Kohlenstoffatomen, beispielsweise Methanol oder Ethanol.

Das erfindungsgemäße Verfahren wird in einem Temperaturbereich von 0 bis 40 °C, vorzugsweise zwischen + 10 und + 30 °C durchgeführt.

Im allgemeinen setzt man 1 Mol der Verbindung (II) mit 1,0 bis 1,2 Mol, vorzugsweise 1,0 bis 1,1 Mol Hydrazin um. Die Reaktionsdauer ist von der Temperatur abhängig und liegt zwischen 2 und 10 Stunden.

Entsprechend dem Reaktionsschema [c] wird in einer zweiten Stufe des Verfahrens die erhaltene Verbindung der allgemeinen Formel (VIII) mit einem Silylierungsreagenz umgesetzt.

Als Silylierungsreagenzien eignen sich vor allem solche Silylverbindungen, die nach Übertragung der Silylgruppe auf die Verbindung (VIII) im weiteren Verlauf der Stufenfolge für weitere Reaktionspartner inert sind, so daß sich eine Isolierung von (IX) erübrigt. Dazu gehören beispielsweise mono- oder bissilylierte Niedrialkancarbonsäureamide wie Bis-(trimethylsilyl)-acetamid oder N-Methyl-N-trimethylsilylacetamid oder Trimethylsilylacetamid, insbesondere N-Methyl-N-trimethylsilylacetamid.

Als Verdünnungsmittel kommen vor allem für die Reaktionspartner inerte organische Lösungsmittel in Frage. Geeignete Lösungsmittel sind vor allem Kohlenwasserstoffe wie Halogenkohlenwasserstoffe, beispielsweise Tetrachlormethan oder Chloroform, oder aromatische Kohlenwasserstoffe, beispielsweise Toluol, cyclische Ether, beispielsweise Tetrahydrofuran oder Dioxan, oder Alkancarbonsäurenitrile mit 1 bsi 4 Kohlenstoffatomen, beispielsweise Acetonitril oder Proprionitril, vorzugsweise Acetonitril.

Das erfindungsgemäße Verfahren wird in einem Temperaturbereich zwischen + 20 und + 80 °C, vorzugsweise zwischen + 30 und + 70 °C durchgeführt.

Im allgemeinen setzt man 1 Mol der Verbindung (VIII) mit 1 bis 3 Mol Silylierungsreagenz, vorzugsweise mit 1 bis 2 Mol um. Ein größerer Überschuß schadet nicht.

Die Reaktionsdauer ist von der Temperatur abhängig und liegt zwischen 15 Minuten und 2 Stunden.

Es ist nicht notwendig, die Verbindung (IX) zu isolieren, etwa durch Abdampfen des Verdünnungsmittels, sondern sie kann im geeigneten Verdünnungsmittel direkt weiter umgesetzt werden.

Entsprechend dem Reaktionsschema [C] wird anschließend die erhaltene Verbindung der allgemeinen Formel (IX) mit einem Trialkylsilylhalogenid der Formel (III) entsprechend der ersten Stufe im Reaktionsschema [A] umgesetzt und das erhaltene silylierte Phosphonoameisensäurehydrazid entsprechend der zweiten Stufe im Reaktionsschema [A] mit Wasser umgesetzt und das erhaltene Phosphonoameisensäurehydrazid beispielsweise in Form seiner Salze, etwa in Form seiner Alkalisalze, etwa durch Versetzen mit einer ausreichenden Menge wäßriger Natronlauge und Eindampfen isoliert.

Die erfindungsgemäßen Phosphonoameisensäurehydrazide wirken gegen Viren, insbesondere gegen Herpes-Viren.

Die neuen Wirkstoffe können als solche oder in Form physiologisch unbedenklicher Salze, beispielsweise von Salzen mit organischen Aminen wie Triethylamin, Cyclohexylamin oder Triethanolamin, oder von Salzen mit anorganischen Kationen, beispielsweise Lithium, Natrium, Kalium, Magnesium, Calcium, Zink oder Ammonium eingesetzt werden.

Die neuen erfindungsgemäßen Wirkstoffe können je nach Verwendungsart beispielsweise lokal, parental oder oral verabreicht werden.

Als Zubereitungsform kommen die üblichen galenischen Applikationsformen in Frage, beispielsweise Cremes, Tabletten, Emulsionen, Infusions- und Injektionslösungen.

Besonders eignen sich 0,1 bis 10 %ige Formulierungen, vor allem wäßrige Lösungen, z.B. gepufferte Lösungen mit einem pH von 6 bis 8.

Als Dosierungen kommen 10 mg bis 1 000 mg/kg Körpergewicht in Frage.

Beispiel 1

Phosphonoameisensäuremethylester, $Na_2$-Salz

22,5 Gewichtsteile Natriumjodid werden in 90 Volumenteilen Acetonitril gelöst und bei 25 °C mit 12,6 Gewichtsteilen Dimethoxyphosphonoameisensäuremethylester versetzt. In die Lösung läßt man bei 25 °C unter Kühlung 17,0 Gewichtsteile Trimethylchlorsilan einfließen, rührt anschließend hintereinander 30 Minuten bei 25 °C und 15 Minuten bei 40 °C, entfernt dann das ausgefallene Natriumchlorid durch Absaugen oder Zentrifugieren und dampft die Lösung im Vakuum ein. Der Abdampfrückstand wird in 30 Volumenteilen Wasser aufgenommen, die erhaltene wäßrige Lösung mit verdünnter Natronlauge neutralisiert und anschließend im Vakuum eingedampft. Nach dem Verreiben des Rückstandes mit Aceton und Isolierung des Kristallisates durch Absaugen erhält man 14,8 Gewichtsteile Phosphonoameisensäuremethylester, $Na_2$-Salz als Hydrat (Ausbeute 90 %).

Beispiel 2

Phosphonoameisensäurehydrazid, $Na_2$-Salz

36,8 Gewichtsteile Phosphonoameisensäuremethylester, $Na_2$-Salz werden in 280 Volumenteilen Methanol suspendiert, bei + 5 bis + 10 °C mit 40,0 Gewichtsteilen Hydrazinhydrat und soviel Wasser versetzt, daß eine Lösung entsteht. Man rührt hintereinander 2 Stunden bei 25 °C und 4 Stunden bei 50 °C, läßt abkühlen und saugt das ausgefallene Reaktionsprodukt ab. Nach Waschen mit Methanol erhält man 30,0 Gewichtsteile Phosphonoameisensäurehydrazid, $Na_2$-Salz (Ausbeute 81 %). $^{13}C$-NMR($D_2O$) $\delta = 178{,}3$ ppm (Dublett, D=O) ; $J_{P,C} = 190$ HZ.

Beispiel 3

Diethoxyphosphonoameisensäurehydrazid

19,6 Gewichtsteile Diethoxyphosphonoameisensäuremethylester werden mit 10 Volumenteilen Ethanol verdünnt und bei + 10 bis + 20 °C mit einer Lösung von 5,0 Gewichtsteilen Hydrazinhydrat in 10 Volumenteilen Ethanol versetzt. Man rührt 8 Stunden bei Raumtemperatur, entfernt das Lösungsmittel im Vakuum und erhält 19,4 Gewichtsteile (Ausbeute 99 %) rohes Diethoxyphosphonoameisensäurehydrazid, das ohne Reinigung weiterverarbeitet wird.

Beispiel 4

Phosphonoameisensäurehydrazid, $Na_2$-Salz

In eine Lösung von 9,7 Gewichtsteilen Diethoxyphosphonoameisensäurehydrazid in 90 Volumenteilen wasserfreiem Acetonitril läßt man bei Raumtemperatur 18,0 Gewichtsteile N-trimethylsilyl-N-methylacetamid fließen, rührt nacheinander 15 Minuten bei 50° und 5 Minuten bei 70 °C, kühlt dann auf 20 °C ab und versetzt das Reaktionsgemisch bei dieser Temperatur mit 15 Gewichtsteilen Natriumjodid. Anschließend läßt man eine Lösung von 12 Gewichtsteilen Trimethyl-chlorsilan in 30 Volumenteilen Acetonitril einfließen, rührt nacheinander 30 Minuten bei 25 °C und 2 Stunden bei 40-45 °C, kühlt dann auf 25 °C ab, entfernt das ausgefallene Natriumchlorid durch Absaugen oder Zentrifugieren und dampft die Lösung im Vakuum ein.

Der Abdampfrückstand wird in 30 Volumenteilen Wasser aufgenommen und die erhaltene wäßrige

Lösung sofort mit verdünnter Natronlauge neutralisiert und anschließend im Vakuum eingedampft. Nach dem Verreiben des Rückstandes mit Methanol und Isolierung des Kristallisates durch Absaugen erhält man 6,6 Gewichtsteile Phosphonoameisensäurehydrazid, $Na_2$-Salz, mit den im Beispiel 2 beschriebenen analytischen Daten (Ausbeute : 72 %).

Beispiel 5

Phosphonoameisensäurephenylester, $Na_2$-Salz

60 Gewichtsteile Natriumiodid werden in 360 Volumenteilen Acetonitril gelöst und bei 25 °C mit 51,6 Gewichtsteilen Diethoxyphosphonoameisensäurephenylester versetzt. In diese Lösung läßt man bei 25 °C unter Kühlung 48 Gewichtsteile Trimethylchlorsilan einfließen, rührt anschließend hintereinander 30 Minuten bei 25 °C und 15 Minuten bei 40 °C, entfernt dann das ausgefallene Natriumchlorid durch Absaugen oder Zentrifugieren und dampft die Lösung im Vakuum ein.

Der Abdampfrückstand wird in 120 Volumenteilen Wasser aufgenommen, die erhaltene Lösung nach Abtrennung des entstandenen Hexamethyldisiloxans mit verdünnter Natronlauge neutralisiert und anschließend im Vakuum eingedampft. Nach dem Verreiben des Rückstandes mit Aceton und Isolierung des Kristallisats durch Absaugen erhält man 45,3 Gewichtsteile Phosphonoameisensäurephenylester, $Na_2$-Salz (Ausbeute 92 %).

Beispiel 6

Phosphonoameisensäurehydrazid, $Na_2$-Salz

24,6 Gewichtsteile Phosphonoameisensäurephenylester, $Na_2$-Salz werden in 70 Volumenteilen Wasser gelöst und bei + 15° bis + 20 °C mit 15,0 Gewichtsteilen Hydrazinhydrat versetzt. Man rührt eine Stunde bei 25 °C und 3 Stunden bei 65 °C, läßt abkühlen, entfernt das entstandene Phenol durch Extraktion mit Ether und dampft die wäßrige Lösung im Vakuum ein. Nach dem Verreiben des Rückstandes mit Methanol und Isolierung des Kristallisats durch Absaugen erhält man 18,4 Gewichtsteile Phosphonoameisensäurehydrazid, $Na_2$-Salz (Ausbeute 100 %).

Beispiel 7

Phosphonoameisensäurephenylester, $Li_2$-Salz

25,8 Gewichtsteile Diethoxyphosphonoameisensäurephenylester werden in 80 Volumenteilen Acetonitril gelöst und bei 20 °C mit 36,7 Gewichtsteilen Trimethylbromsilan versetzt. Man rührt 2 Stunden bei 40-45 °C und dampft anschließend die Lösung im Vakuum ein. Der Abdampfrückstand wird in 60 Volumenteilen Wasser aufgenommen, die erhaltene Lösung nach Abtrennung des entstandenen Hexamethyldisiloxans mit verdünnter Lithiumlauge neutralisiert und anschließend im Vakuum eingedampft.

Nach dem Verreiben des Rückstandes mit Aceton und Isolierung des Kristallisats durch Absaugen erhält man 20 Gewichtsteile Phosphonoameisensäurephenylester, $Li_2$-Salz (Ausbeute 93 %).

Beispiel 8

Phosphonoameisensäurehydrazid, $Li_2$-Salz

8,6 Gewichtsteile Phosphonoameisensäurephenylester, $Li_2$-Salz werden in 86 Volumenteilen Wasser verteilt und bei 20 °C mit 8,0 Gewichtsteilen Hydrazinhydrat versetzt. Man rührt eine Stunde bei 25 °C und 3 Stunden bei 60 °C, läßt abkühlen, entfernt das entstandene Phenol durch Extraktion mit Ether und dampft die wäßrige Lösung im Vakuum ein. Nach dem Verreiben des Rückstands mit Methanol und Isolierung des Kristallisats durch Absaugen erhält man 5,5 Gewichtsteile Phosphonoameisensäurehydrazid, $Li_2$-Salz (Ausbeute 90 %).

**Ansprüche**

1. Phosphonoameisensäurehydrazide der Formel

$$R\text{-}NH\text{-}NH\text{-}CO\text{-}\overset{O}{\overset{\|}{P}}(OH)_2 \qquad (I)$$

in der R für Wasserstoff oder Alkyl mit 1-6 C-Atomen steht, und/oder physiologisch verträgliche Salze derselben.

2. Verfahren zur Herstellung von Phosphonoameisensäurehydraziden der Formel (I) und/oder deren physiologisch verträglichen Salze, dadurch gekennzeichnet, daß man entweder

a) einen Phosphonoameisensäure ester der Formel (II)

$$R_2O\text{-}CO\text{-}\overset{O}{\overset{\|}{P}}(O\text{-}R_1)_2 \qquad (II)$$

in welcher $R_1$ für Alkyl steht und $R_2$ für Alkyl und Aryl steht, mit einem Trialkylsilyhalogenid der Formel (III)

$$(R_3)_3Si\text{—}Hal \qquad (III)$$

in der $R_3$ für einen Alkylrest und Hal für Brom oder Jod stehen, umsetzt, die erhaltene Verbindung der Formel (IV)

$$R_2O\text{-}CO\text{-}\overset{O}{\overset{\|}{P}}(O\text{-}Si(R_3)_3)_2 \qquad (IV)$$

in der $R_2$ und $R_3$ die obige Bedeutung haben, mit Wasser umsetzt und die erhaltene Verbindung der Formel (V)

$$R_2O\text{-}CO\text{-}\overset{O}{\overset{\|}{P}}(OH)_2 \qquad (V)$$

in der $R_2$ die obige Bedeutung hat, in Form ihrer Salze, beispielsweise ihrer Alkalisalze, mit einem Hydrazin der Formel (VI)

$$R\text{—}NH\text{—}NH_2 \qquad (VI)$$

in der R die obige Bedeutung hat, umsetzt, oder

b) einen Phosphonoameisensäureester der Formel (Vii)

$$R_2O\text{-}CO\text{-}\overset{O}{\overset{\|}{P}}(O\text{-benzyl})_2 \qquad (VII)$$

in welcher $R_2$ die obige Bedeutung hat, mit Wasserstoff in Gegenwart eines Edelmetallkatalysators die Benzylgruppe hydrogenolytisch abspaltet, die erhaltene Verbindung der Formel (V), in der $R_2$ die obige Bedeutung hat, in Form ihrer Salze, beispielsweise ihrer Alkalisalze, mit einem Hydrazin der Formel (VI) umsetzt, oder

c) Phosphonoameisensäureester der Formel (II) mit einem Hydrazin umsetzt, die erhaltene Verbindung der Formel (VIII)

$$R\text{-}NH\text{-}NH\text{-}CO\text{-}\overset{O}{\overset{\|}{P}}(O\text{-}R_1)_2 \qquad (VIII)$$

in der R und $R_1$ obige Bedeutungen haben, mit einem Silylierungsreagens umsetzt und die erhaltene Verbindung der Formel (IX)

$$(R_3)_3Si\text{-}\overset{R}{\overset{|}{N}}\text{-}NH\text{-}CO\text{-}\overset{O}{\overset{\|}{P}}(OR)_1)_2 \qquad (IX)$$

in der R, $R_1$ und $R_3$ obige Bedeutungen haben, mit einem Trialkylsilylhalogenid der Formel (III) umsetzt und die erhaltene Verbindung der Formel (X)

$$\overset{\displaystyle R}{\underset{|}{}}\;\;(R_3)_3Si\text{-}\overset{R}{\overset{|}{N}}\text{-}NH\text{-}CO\text{-}\overset{O}{\overset{\|}{P}}(O\text{-}Si(R_3)_3)_2 \qquad (X)$$

in der R und $R_3$ die obige Bedeutung haben, mit Wasser umsetzt, und die so erhaltenen Verbindungen gegebenenfalls in ihre physiologisch verträglichen Salze überführt.

3. Verfahren nach Anspruch 2, dadurch gekennzeichnet, daß man als Phosphonoameisensäurealkylester Formel II solche verwendet, in denen vorzugsweise $R_1$ für Alkyl mit 1 bis 4 Kohlenstoffatomen, insbesondere für Methyl und Ethyl, und $R_2$ für Alkyl mit 1 bis 2 Kohlenstoffatomen, insbesondere für Methyl oder für Aryl, insbesondere für Phenyl steht.

4. Verfahren nach Anspruch 2, dadurch gekennzeichnet, daß man als Trialkylsilylhalogenide der Formel III solche verwendet, in denen vorzugsweise $R_3$ für Alkyl mit 1 bis 4 Kohlenstoffatomen, insbesondere für Methyl, und Hal für Brom oder Jod steht.

5. Verfahren nach Anspruch 2, dadurch gekennzeichnet, daß man als Hydrazine der Formel VI solche verwendet, in denen R für Wasserstoff oder Alkyl mit 1 bis 6 Kohlenstoffatomen, insbesondere mit 1 bis 4 Kohlenstoffatomen steht.

6. Arzneimittel, gekennzeichnet durch einen Gehalt an mindestens einem Phosphonoameisensäurehydrazid und/oder deren physiologisch verträglichen Salze gemäß Anspruch 1.

7. Verfahren zur Herstellung von antiviralen Mitteln, dadurch gekennzeichnet, daß man Phosphonoameisensäurehydrazid gemäß Anspruch 1 mit inerten, nichttoxischen, pharmazeutisch geeigneten Trägerstoffen vermischt.

**Claims**

1. Phosphonoformic acid hydrazides of the formula

$$R\text{-}NH\text{-}NH\text{-}CO\text{-}\overset{O}{\overset{\|}{P}}(OH)_2 \qquad (I)$$

in which R represents hydrogen or alkyl with 1-6 C atoms, and/or physiologically acceptable salts thereof.

2. Process for the production of phosphonoformic acid hydrazides of the formula (I) and/or physiologycally acceptable salts thereof, characterised in that either

a) a phosphonoformic acid ester of the formula (II)

$$R_2O\text{-}CO\text{-}\overset{O}{\overset{\|}{P}}(O\text{-}R_1)_2 \qquad (II)$$

in which $R_1$ represents alkyl and $R_2$ represents alkyl and aryl, is reacted with a trialkylsilyl halide of the formula (III)

$$(R_3)_3Si\text{—}Hal \qquad (III)$$

in which $R_3$ represents an alkyl radical and Hal represents bromine or iodine, the resulting compound of the formula (IV)

$$R_2O\text{-}CO\text{-}\overset{O}{\overset{\|}{P}}(O\text{-}Si(R_3)_3)_2 \qquad (IV)$$

in which $R_2$ and $R_3$ have the above meaning, is reacted with water and the resulting compound of the formula (V)

$$R_2O\text{-}CO\text{-}\overset{O}{\overset{\|}{P}}(OH)_2 \qquad (V)$$

in which $R_2$ has the above meaning, in the form of salts thereof, for example alkali metal salts thereof, is

reacted with a hydrazine of the formula (VI)

$$R—NH—NH_2 \qquad (VI)$$

in which R has the above meaning, or

b) a phosphonoformic acid ester of the formula (VII)

$$R_2O-CO-\overset{O}{\overset{\parallel}{P}}(O\text{-benzyl})_2 \qquad (VII)$$

in which $R_2$ has the above meaning, the benzyl group is split off hydrogenolytically with hydrogen in the presence of a noble metal catalyst, and the resulting compound of the formula (V), in which $R_2$ has the above meaning, in the form of salts thereof, for example alkali metal salts thereof, is reacted with a hydrazine of the formula (VI), or

c) a phosphonoformic acid ester of the formula (II) is reacted with a hydrazine, the resulting compound of the formula (VIII)

$$R-NH-NH-CO-\overset{O}{\overset{\parallel}{P}}(O-R_1)_2 \qquad (VIII)$$

in which R and $R_1$ have the above meanings, is reacted with a silylating reagent and the resulting compound of the formula (IX)

$$(R_3)_3Si-\overset{R}{\overset{|}{N}}-NH-CO-\overset{O}{\overset{\parallel}{P}}(OR)_1)_2 \qquad (IX)$$

in which R, $R_1$ and $R_3$ have the above meanings, is reacted with a trialkylsilyl halide of the formula (III) and the resulting compound of the formula (X)

$$(R_3)_3Si-\overset{R}{\overset{|}{N}}-NH-CO-\overset{O}{\overset{\parallel}{P}}(O-Si(R_3)_3)_2 \qquad (X)$$

in which R and $R_3$ have the above meaning, is reacted with water, and the resulting compounds are optionally converted into their physiologically acceptable salts.

3. Process according to Claim 2, characterized in that the phosphonoformic acid alkyl esters of the formula II employed are those in which preferably $R_1$ represents alkyl with 1 to 4 carbon atoms, in particular methyl and ethyl, and $R_2$ represents alkyl with 1 to 2 carbon atoms, in particular methyl, or aryl, preferably phenyl.

4. Process according to Claim 2, characterised in that the trialkylsilyl halides of the formula III employed are those in which preferably $R_3$ represents alkyl with 1 to 4 carbon atoms, in particular methyl, and Hal represents bromine or iodine.

5. Process according.to Claim 2, characterized in that the hydrazines of the formula VI employed are those in which R represents hydrogen or alkyl with 1 to 6 carbon atoms, in particular with 1 to 4 carbons atoms.

6. Medicaments, characterized in that they contain at least one phosphonoformic acid hydrazide, and/or physiologically acceptable salts thereof, according to Claim 1.

7. Process for the preparation of antiviral agents, characterised in that phosphonoformic acid hydrazide according to Claim 1 is mixed with inert, non-toxic, pharmaceutically suitable excipients.

**Revendications**

1. Phosphonoformhydrazides de formule

$$R-NH-NH-CO-\overset{O}{\overset{\parallel}{P}}(OH)_2 \qquad (I)$$

dans laquelle R représente l'hydrogène ou un groupe alkyle en $C_1$-$C_6$, et/ou leurs sels physiologiquement compatibles.

2. Procédé pour la préparation de phosphonoformhydrazides de formule (I) et/ou de leurs sels physiologiquement compatibles, caractérisé en ce que

a) on fait réagir un ester d'acide phosphonoformique de formule

$$R_2O\text{-}CO\text{-}\overset{\overset{O}{\parallel}}{P}(O\text{-}R_1)_2 \qquad (II)$$

dans laquelle $R_1$ représente un groupe alkyle et $R_2$ représente un groupe alkyle ou aryle, avec un halogénure de trialkysilyle de formule

$$(R_3)_3Si\text{—}Hal \qquad (III)$$

dans laquelle $R_3$ représente un reste alkyle et Hal représente le brome ou l'iode, on fait réagir le composé obtenu de formule

$$R_2O\text{-}CO\text{-}\overset{\overset{O}{\parallel}}{P}(O\text{-}Si(R_3)_3)_2 \qquad (IV)$$

dans laquelle $R_2$ et $R_3$ ont la signification ci-dessus, avec l'eau, et on fait réagir le composé obtenu de formule

$$R_2O\text{-}CO\text{-}\overset{\overset{O}{\parallel}}{P}(OH)_2 \qquad (V)$$

dans laquelle $R_2$ a la signification ci-dessus, sous forme de ses sels, par exemple de ses sels alcalins, avec une hydrazine de formule

$$R\text{—}NH\text{—}NH_2 \qquad (VI)$$

dans laquelle R a la signification ci-dessus, ou bien

b) on élimine par hydrogénolyse le groupe benzyle d'un phosphonoformiate de formule

$$R_2O\text{-}CO\text{-}\overset{\overset{O}{\parallel}}{P}(O\text{-}benzyl)_2 \qquad (VII)$$

dans laquelle $R_2$ a la signification ci-dessus, par l'hydrogène en présence d'un catalyseur à base de métal noble, on fait réagir le composé obtenu de formule (VI), dans laquelle $R_2$ a la signification ci-dessus, sous forme de ses sels, par exemple ses sels alcalins, avec une hydrazine de formule (VI), ou bien

c) on fait réagir un phosphonoformiate d'alkyle de formule (II) avec une hydrazine, on fait réagir le composé obtenu de formule

$$R\text{-}NH\text{-}NH\text{-}CO\text{-}\overset{\overset{O}{\parallel}}{P}(O\text{-}R_1)_2 \qquad (VIII)$$

dans laquelle R et $R_1$ ont les significations ci-dessus, avec un réactif de silylation, et on fait réagir le composé obtenu de formule

$$(R_3)_3Si\text{-}\underset{}{\overset{\overset{R}{|}}{N}}\text{-}NH\text{-}CO\text{-}\overset{\overset{O}{\parallel}}{P}(OR)_1)_2 \qquad (IX)$$

dans laquelle R, $R_1$ et $R_3$ ont les significations ci-dessus, avec un halogénure de trialkylsilyle de formule

(III) et on fait réagir avec l'eau le composé obtenu de formule

$$(R_3)_3Si-\overset{\overset{R}{|}}{N}-NH-CO-\overset{\overset{O}{\|}}{P}(O-Si(R_3)_3)_2 \qquad (X)$$

dans laquelle R, $R_1$ et $R_3$ ont la signification ci-dessus, et on transforme éventuellement les composés ainsi obtenus en leurs sels physiologiquement acceptables.

3. Procédé selon la revendication 2, caractérisé en ce que l'on utilise comme phosphonoformiates d'alkyle de formule (II) ceux dans lesquels $R_1$ représente de préférence un groupe alkyle en $C_1$-$C_4$, en particulier méthyle et éthyle, et $R_2$ un groupe alkyle en $C_1$-$C_2$, en particulier méthyle, ou un groupe aryle, en particulier phényle.

4. Procédé selon la revendication 2, caractérisé en ce que l'on utilise comme halogénures de trialkylsilyle de formule (III) ceux dans lesquels $R_3$ représente de préférence un groupe alkyle en $C_1$-$C_4$, en particulier méthyle, et Hal le brome ou l'iode.

5. Procédé selon la revendication 2, caractérisé en ce que l'on utilise comme hydrazines de formule (VI) celles dans lesquelles R représente l'hydrogène ou un groupe alkyle en $C_1$-$C_6$, en particulier en $C_1$-$C_4$.

6. Médicaments, caractérisés en ce qu'ils contiennent au moins un phosphonoformhydrazide et/ou un de leurs sels physiologiquement compatibles selon la revendication 1.

7. Procédé pour la préparation d'agent antiviraux, caractérisé en ce que l'on mélange un phosphonoformhydrazide selon la revendication 1, avec des supports inertes non toxiques appropriés en pharmacie.